# EUROPÄISCHE PATENTSCHRIFT

(11) **EP 1 843 725 B1**
(45) Veröffentlichungstag und Bekanntmachung des Hinweises auf die Patenterteilung: **23.11.2011**
(21) Anmeldenummer: 06705920.4
(22) Anmeldetag: 01.02.2006
(51) Int. Cl.: A61F 9/008, A61B 3/103

(54) **VORRICHTUNG ZUM MESSEN UND ZUR CHIRURGISCHEN KORREKTUR VON ABBILDUNGSFEHLERN IM MENSCHLICHEN AUGE**
DEVICE FOR MEASURING AND FOR SURGICAL CORRECTION OF IMAGING ERRORS IN THE HUMAN EYE
DISPOSITIF POUR MESURER ET CORRIGER DE FACON CHIRURGICALE DES DEFAUTS DE REPRESENTATION DANS L'OEIL HUMAIN

(30) Priorität: 01.02.2005 DE 102005005564
(43) Veröffentlichungstag der Anmeldung: 17.10.2007
(73) Patentinhaber: Heiberger, Kurt, 90455 Nürnberg (DE)
(72) Erfinder: Heiberger, Kurt, 90455 Nürnberg (DE)
(74) Vertreter: Heimler, Rainer
(86) Internationale Anmeldenummer: PCT/DE2006/000192
(87) Internationale Veröffentlichungsnummer: WO 2006/081814

(56) Entgegenhaltungen:
- EP-A- 1 231 496
- DE-A1- 10 207 535
- US-A1- 2003 179 344

## Beschreibung

Die Erfindung betrifft eine Vorrichtung zum Messen und zur chirurgischen Korrektur von Abbildungsfehlern im menschlichen Auge, bei der das optische System des Auges mit einem Lichtstrahl einer Laserdiode flächendeckend abtastbar und die reflektierten Lichtstrahlen messbar und auswertbar sind, wobei in den Mess- bzw. Abbildungsstrahlengang zwischen der Laserdiode und dem Auge ein erster Strahlteiler eingefügt ist, über den ein Lichtstrahl eines Bearbeitungslasers für eine chirurgische Korrektur der Hornhaut und/oder der Linse und/oder der Netzhaut des Auges einkoppelbar ist, wobei ferner in den Messstrahlengang zwischen der Laserdiode und dem ersten Strahlteiler zum flächendeckenden Abtasten des optischen Systems des Auges ein zweidimensional ablenkbarer Kippspiegel eingefügt ist.

Aus der US 2003/0179344 A1 ist eine Vorrichtung der eingangs erwähnten Art bekannt, die Teil einer ophthalmologischen Laserstrahlabtastung der Netzhaut ist, wobei die Abtastung für eine selektive Mikrokoagulation der Netzhaut mit minimalen optischen Aberrationen optimiert ist.

Aus der EP 1 231 496 A2 ist eine Vorrichtung der eingangs erwähnten Art bekannt, die Teil einer ophthalmologischen Operationsvorrichtung ist und mit einer optischen Kohärenztomographie-Vorrichtung arbeitet, um Abschnitte des optischen Systems zu bestimmen, die durch die Anwendung der Laserstrahlen beeinflusst worden sind.

Aus der DE 102 07 535 A1 ist eine Vorrichtung der eingangs erwähnten Art bekannt, bei der jedoch der Messstrahl und der Bearbeitungsstrahl nicht aus zwei getrennten Laserdioden stammen, sondern es wird der Lichtstrahl einer einzigen Laserdiode mit einem Strahlenteiler in einen Messstrahl und einen Bearbeitungsstrahl aufgeteilt. Der zeitliche Zusammenhang, insbesondere die Kohärenzbeziehung der beiden Strahlen liefert eine Abstandsinformation, die die Obeflächenstruktur des bearbeiteten Messobjektes beschreibt.

Bei dem bekannten Stand der Technik besteht in keinem Fall die Möglichkeit zum Vorkompensieren und zum Anpassen des Messstrahles an die mittlere Brechkraft des zu messenden Auges, so dass eine optimale Fokussierung des Messstrahles nicht möglich ist.

Der Erfindung liegt die Aufgabe zugrunde, eine Vorrichtung der eingangs erwähnten Art so zu verbessern, dass es ermöglicht wird, während einer Vermessung und Behandlung des Auges mit einem Lasersystem das Behandlungsergebnis mit optimaler Genauigkeit zu überprüfen.

Diese Aufgabe wird durch eine Vorrichtung mit den Merkmalen des Anspruchs 1 gelöst. Weitere vorteilhafte Ausgestaltungen der Erfindung sind in den Unteransprüchen angegeben.

Die mit der Erfindung erzielten Vorteile bestehen insbesondere darin, dass durch die optimale Fokussierung des Messstrahles während der chirurgischen Behandlung des Auges das Ergebnis jedes einzelnen Korrekturschrittes sofort genauestens abgelesen und, wenn nötig, gleichzeitig nachgebessert werden kann.

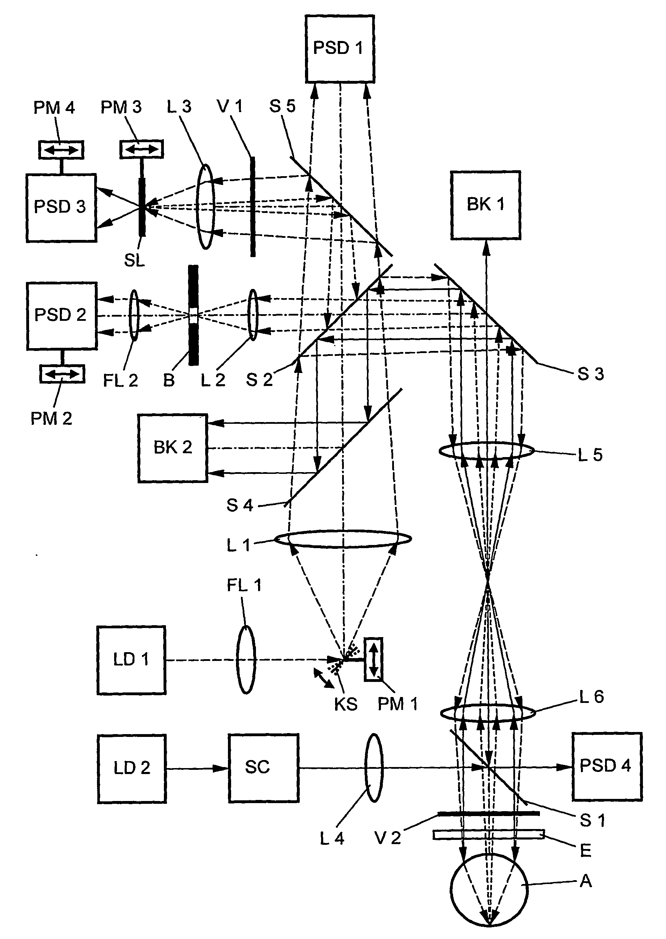

Ein Ausführungsbeispiel der Erfindung ist in der Zeichnung dargestellt und wird im folgenden näher beschrieben. Sie zeigt den Mess-, Abbildungs- und Bearbeitungsstrahlengang einer erfindungsgemäßen Vorrichtung.

Zum Messen von Abbildungsfehlern, auch von solchen höherer Ordnung, im menschlichen Auge A wird dessen optisches System mit einem Messstrahl einer Laserdiode LD 1 flächendeckend abgetastet. Die reflektierten Abbildungsstrahlen werden gemessen und ausgewertet, um aufgrund des Ergebnisses einen chirurgischen Eingriff an dem Auge A vorzunehmen. Hierfür ist in den Mess- bzw. Abbildungsstrahlengang zwischen der Laserdiode LD 1 und dem Auge A ein Strahlteiler S 1 eingefügt, über den ein Bearbeitungsstrahl eines Bearbeitungslasers LD 2 für eine chirurgische Korrektur der Hornhaut und/oder der Linse und/oder der Netzhaut des Auges A einkoppelbar ist.

Zum Abtasten des Auges A ist zwischen der Laserdiode LD 1 und dem Strahlteiler S 1 ein Miniatur-Kippspiegel KS in Form eines 2 D Mikro-Scanner-Spiegels in den Messstrahlengang eingefügt, mit dem der Messstrahl zum flächendeckenden Abtasten des Auges A mit Frequenzen von 150 Hz bis 32 kHz zweidimensional ablenkbar ist.

Um über den gesamten Messbereich eine gleichbleibend hohe Messgenauigkeit zu gewährleisten, wird die Divergenz des von der Laserdiode LD 1 gelieferten Laserstrahls mit einer elektrisch steuerbaren Flüssigkeitslinse FL 1 in Abhängigkeit von der zu messenden Fehlsichtigkeit des Auges eingestellt, wodurch ein gleichbleibender Strahldurchmesser von weniger als 200 µm sichergestellt ist.

Zwischen dem Kippspiegel KS und dem ersten Strahlteiler S 1 ist eine Linse L 1 in den Messstrahlengang eingefügt, deren Abstand zu dem Kippspiegel KS zum Vorkompensieren und zum Anpassen des Messstrahles an die mittlere Brechkraft des Auges A durch einen Piezomotor PM 1 veränderbar ist. Dabei wird der Kippspiegel KS je nach der gemessenen Brechkraft entweder im Brennpunkt, vor dem Brennpunkt oder hinter dem Brennpunkt der Linse L 1 stehen. Die axiale Verschiebung durch den Piezomotor PM 1 positioniert den Kippspiegel im nm-Bereich, so dass die Vorkompensation des Messstrahles im Bereich von weniger als 0,1 dpt erfolgt.

Ferner ist zwischen der Linse L 1 und dem Strahlteiler S 1 ein weiterer Strahlteiler S 2 in den Mess- bzw. Abbildungsstrahlengang eingefügt, der einen Teil des Messstrahles auskoppelt und einem Positionsdetektor PSD 1, der durch eine schnelle, empfindliche Kamera ersetzt werden kann, zuführt, der den gleichen Abstand von dem Strahlteiler S 2 hat wie das Auge A (in der Zeichnung nicht maßstabgerecht dargestellt) und die Position des auf der Hornhaut des Auges A auftreffenden Messstrahles erfasst. Gleichzeitig werden durch den Positionsdetektor PSD 1 die Laserleistung und die Laserenergie des Messstrahles überwacht. Der Messstrahlengang ist in der Zeichnung großgestrichelt dargestellt.

Im Folgenden wird der Abbildungsstrahlengang beschrieben, der in der Zeichnung kleingestrichelt dargestellt ist. Zwischen den Strahlteilern S 1 und S 2 ist ein weiterer Strahlteiler S 3 in den Mess- bzw. Abbildungsstrahlengang eingefügt, der den Strahlengang der von der Netzhaut des Auges A gestreuten Abbildungsstrahlen aufteilt. Dabei gelangt zum Messen der Brechkraft des optischen Systems des Auges A der erste Teil der Abbildungsstrahlen über eine weitere Linse L 2 und eine den Raumwinkel des Abbildungsstrahlenganges begrenzenden Blende B sowie über eine elektrisch steuerbare Flüssigkeitslinse FL 2 zu einem weiteren Positionsdetektor PSD 2 oder zu einer schnellen Kamera mit oder ohne Bildverstärker. Die Blende B lässt nur das Licht durch, welches das Auge A entlang der optischen Achse (Sehachse) verlässt. Die lokale Brechkraft des Auges A auf der optischen Achse wird als Bezugspunkt definiert. Die Ist-Position des empfangenen Messstrahles wird mit der Soll-Position verglichen. Die Abweichung von der Soll-Position ist ein Maß für die lokale Brechkraft des zu messenden Auges A. Der Abbildungsstrahl ist durch eine entsprechende Steuerung der Flüssigkeitslinse FL 2 an den Positionsdetektor PSD 2 bzw. an die Kamera anpassbar.

Der zweite Teil der Abbildungsstrahlen gelangt zu einer Beobachtungskamera BK 1, die mit einem Gerät zum Aufzeichnen von Augen- bzw. Blickbewegungen einer Testperson (Eye Tracking) ausgerüstet oder wahlweise durch andere optische Geräte ersetzbar ist, wie beispielsweise durch ein Mikroskop oder ein Schichtdickenmessgerät.

Um über den gesamten Messbereich eine gleichbleibend hohe Messgenauigkeit zu gewährleisten, wird der Positionsdetektor PSD 2 als Messempfänger mit einem Piezomotor PM 2 an diejenige Position gestellt, die je nach zu messender mittlerer Brechkraft eine konstante Bildgröße und damit eine gleichbleibende Auflösung sicherstellt. Bei Verwendung der steuerbaren Flüssigkeitslinse FL 2 kann auf den Piezomotor PM 2 verzichtet werden. Die Flüssigkeitslinse FL 2 gewährleistet eine konstante Bildgröβe und damit eine gleichbleibende Messgenauigkeit über den gesamten Messbereich. Die Dynamik des Messgerätes kann durch Kombination des Piezomotors PM 2 mit der Flüssigkeitslinse FL 2 erhöht werden.

In den Messstrahlengang zwischen der Linse L 1 und dem Strahlenteiler S 2 ist ein weiterer Strahlenteiler S 4 eingefügt, der die an der Oberfläche der Hornhaut des Auges A reflektierten Messstrahlen nach weiteren Reflexionen an den Strahlenteilern S 3 und S 2 zu einer Beobachtungskamera BK 2 mit telezentrischem Objektiv umlenkt, womit der Abstand zwischen der Einblicköffnung E des Messgerätes und dem Auge A gemessen und der Krümmungsradius der Hornhaut des Auges A überprüft wird.

Bevor die Brechkraft des Auges A gemessen wird, muss es fixiert werden, das heißt, es wird in einem entspannten Zustand vermessen. Damit das Auge A nicht akkommodiert, wird mit dem Kippspiegel KS ein blinkender Punkt oder Ring als Sehzeichen eingeblendet, auf das sich das Auge A anpasst. Mit der Flüssigkeitslinse FL 1 und dem Kippspiegel KS lassen sich der Punktdurchmesser bzw. die Ringgröße einstellen. Damit das Auge A die Helligkeit und Größe des Sehzeichens über den ganzen Messbereich gleich empfindet, wird die Lichtleistung des Lasers angepasst und die Bildgröße über die Flüssigkeitslinse FL 1 und den Kippspiegel KS gesteuert.

Mit zeitlich abgestimmten Laserpulsen lassen sich bestimmte Muster auf das Auge A projizieren. Das Muster kann so verzerrt werden, dass unter Berücksichtigung der vorher gemessenen lokalen Brechkraft des Auges A ein symmetrisches Punktemuster mit dem Positionsdetektor PSD 2 gemessen wird. Die Verzerrung des Punktemusters (Koma, Astigmatismus usw.) wird vorher berechnet. Dies kann als Testmessung benutzt werden.

Wird ein technisches Messobjekt vermessen, so wird das Auge A durch ein "Kunstauge" ersetzt, das aus einer Linse L 3, einer Streulichtscheibe SL sowie einem Positionsdetektor PSD 3 oder einer weiteren Kamera besteht. Über einen Strahlteiler S 5, der zwischen dem Strahlteiler S 2 und dem Positionsdetektor PSD 1 in den Messstrahlengang eingefügt ist, wird der Mess- bzw. Abbildungsstrahlengang für das Kunstauge ein- bzw. ausgekoppelt, der mit einem Verschluss V 1 unterbrochen werden kann. Die Streulichtscheibe SL wird mit einem Piezomotor PM 3 in einen definierten Abstand zum Messobjekt gebracht. Der Positionsdetektor PSD 3 kann durch einen Piezomotor PM 4 axial verschoben werden. Das von der Streulichtscheibe SL gestreute Licht wird über den Abbildungsstrahlengang von dem Positionsdetektor PSD 2 eingefangen und ausgewertet. Dabei wird die Position des von der Streulichtscheibe SL gestreuten Messstrahles unter einem kleinen Raumwinkel über die Linse L 2 und die Blende B gemessen, die nur das Licht durchlässt, das die Linse L 3 durch ihre Mitte verlässt. Die lokale Brechkraft der Linse L 3 in der optischen Achse wird als Bezugspunkt definiert. Die Ist-Position des empfangenen Laserstrahles wird mit der Soll-Position verglichen. Die Abweichung von der Soll-Position ist ein Maß für die lokale Brechkraft des zu messenden Kunstauges.

Vor jeder Messung werden durch Selbstkalibrieren alle Strahlengänge überprüft. Ein Verschluss V 2, der an der Einblicköffnung E für das zu messende Auge A angeordnet ist, wird geschlossen, so dass von außen kein Licht in das Messgerät einstrahlen kann. Der vor dem Kunstauge angeordnete Verschluss V 1 wird geöffnet und das Kunstauge gemessen. Vor der Messung wird beim Kunstauge die mittlere Brechkraft des Auges A eingestellt. Das Kunstauge befindet sich im gleichen Abstand zum Positionsdetektor PSD 2 wie das Auge A. Außerdem müssen, wie bei jeder normalen Messung, die Strahlengänge vorkompensiert und der Durchmesser des Laserstrahls eingestellt werden. Das von der Streulichtscheibe SL gestreute Licht wird unter einem kleinen Raumwinkel zum Positionsdetektor PSD 2 geführt. Der Positionsdetektor PSD 3 hinter der Streulichtscheibe SL überwacht auch den Messstrahlengang, so dass ein Fehler im optischen Strahlengang leicht eingegrenzt werden kann. Weicht das Messergebnis von dem erwarteten Wert ab, so ist dies ein Hinweis darauf, dass die Piezomotoren PM 1, PM 2 und PM 3 nicht richtig funktionieren und/oder die Flüssigkeitslinsen FL 1 und FL 2 nicht richtig angesteuert werden.

Während des normalen Messvorganges wird die Streulichtscheibe SL aus dem Strahlengang entfernt, so dass in diesem Fall der Positionsdetektor PSD 3 nur den Messstrahl überwacht.

Die Positionsdetektoren PSD 1 bis PSD 3 sind in ihrer Bandbreite auf 1,6 kHz bis 16 kHz begrenzt. Deshalb werden Entzerrer eingesetzt, die die linearen Verzerrungen kompensieren. Schnelle Abtast-Halteglieder und schnelle Analog/Digital-Wandler ermöglichen es, mit Digital-Signal-Prozessoren die Lichtleistung, die Lichtenergie und die Lage des Laserstrahles auf den Positionsdetektoren so schnell wie möglich zu bestimmen. Der Positionsdetektor PSD 1 berechnet die Lage des Messstrahles auf der Vorderseite des Messobjektes (entsprechend der Hornhaut beim Auge), während der Positionsdetektor PSD 2 die Lage des von der Streulichtscheibe SL (entsprechend der Netzhaut beim Auge) gestreuten Lichtes und damit die lokale Brechkraft des Messobjektes bestimmt, die von dem Positionsdetektor PSD 2 mit der lokalen Sollbrechkraft verglichen wird. Bei Abweichungen von der lokalen Sollbrechkraft werden die Energie, die Anzahl der Laserpulse und die Pulsdauer der Laserpulse für den Bearbeitungslaser LD 2 berechnet und an diesen übertragen, so dass dieser Korrekturen an dem Auge durchführen kann.

Der Bearbeitungsstrahl des Bearbeitungslasers LD 2 wird mit Hilfe zweier Scanner SC in X- bzw. Y-Richtung abgelenkt und mit dem Strahlteiler S 1 in den Mess- bzw. AbbildungsStrahlengang eingekoppelt. Ein weiterer Positionsdetektor PSD 4 überwacht die Energie, die Pulsbreite und die Leistung des Bearbeitungslasers LD 2 und misst gleichzeitig die aktuelle Position des Laserstrahles des Bearbeitungslasers LD 2 und dient zum Justieren des Bearbeitungs-, Mess- bzw. Abbildungsstrahlenganges.

Mit dem Positionsdetektor PSD 4 kann gleichzeitig die Position des Messstrahles auf dem Messobjekt erfasst werden, so dass die Zuordnung des Messergebnisses zur Bearbeitungsstelle sichergestellt ist. Der Positionsdetektor PSD 4 hat den gleichen Abstand vom ersten Strahlteiler S 1 wie das Auge A.

Der Arbeitsabstand des Bearbeitungslasers LD 2 wird über eine Linse L 4 eingestellt. Um den Arbeitsabstand des Bearbeitungslasers LD 2 mit dem Messabstand des Messgerätes abzustimmen, können weitere Linsen L 5 und L 6 mit einer Abbildung im Verhältnis 1:1 zwischen den Strahlteilern S 1 und S 3 in den Mess- bzw. Abbildungsstrahlengang eingefügt werden, um den Messabstand beliebig einstellen zu können.

Eine Vereinfachung der Messvorrichtung ergibt sich dadurch, dass die Blende B, die zweite Flüssigkeitslinse FL 2 und der zweite Positionsdetektor PSD 2 gemeinsam mit der Laserdiode LD 1, der ersten Flüssigkeitslinse FL 1, dem Kippspiegel KS und dem ersten Piezomotor PM 1 auf einem Positioniersystem derart montiert sind, dass die optische Achse des durch die Öffnung der Blende B eintretenden Abbildungsstrahles parallel zu der optischen Achse des den Kippspiegel KS verlassenden Messstrahles verläuft, wobei das Positioniersystem durch einen weiteren Piezomotor in Richtung der optischen Achsen verschiebbar ist.

Eine weitere Vereinfachung der Messvorrichtung lässt sich dadurch erreichen, dass die Blende B, die zweite Flüssigkeitslinse FL 2 sowie der zweite Positionsdetektor PSD 2 gemeinsam mit dem Kippspiegel KS auf einem Positioniersystem derart montiert sind, dass die optische Achse des durch die Öffnung der Blende B eintretenden Abbildungsstrahles parallel zu der optischen Achse des den Kippspiegel KS verlassenden Messstrahles verläuft, wobei der von der Laserdiode LD 1 kommende Messstrahl über dieselbe optische Achse den Kippspiegel KS erreicht, über die der abgelenkte Messstrahl den Kippspiegel KS verlässt, und wobei das Positioniersystem durch einen Piezomotor in Richtung der optischen Achsen verschiebbar ist. Da sich auf dem Positioniersystem nur wenige Bauelemente befinden, kann der Piezomotor zum Antrieb des Positioniersystems wesentlich kleiner sein als in dem vorher beschriebenen Beispiel.

Die Verlegung des ankommenden Messstrahles und des abgelenkten Strahles in dieselbe optische Achse ist dadurch möglich, dass der von der Laserdiode LD 1 kommende Messstrahl über die erste Flüssigkeitslinse FL 1 auf einen Polarisationswürfel trifft, dort reflektiert wird und über eine λ/4-Platte über die optische Achse den Kippspiegel KS erreicht, von wo aus der abgelenkte Messstrahl über die optische Achse, über die λ/4-Platte, über den Polarisationswürfel und über eine Linse auf das zu messende Auge A trifft, von wo aus die an der Netzhaut gestreuten Abbildungsstrahlen über diese Linse auf den Polarisationswürfel treffen, dort reflektiert werden und über eine weitere Linse und einen reflektierenden Strahlenteiler durch die Blende B und die Flüssigkeitslinse FL 2 den Positionsdetektor PSD 2 erreichen, während die an der Hornhaut des Auges A reflektierten Strahlen auf demselben Weg wie die Abbildungsstrahlen auf den Strahlenteiler treffen, dort durchgelassen werden, um über eine weitere Linse die Beobachtungskamera BK 2 mit telezentrischem Objektiv zu erreichen.

Das letzte Beispiel unterscheidet sich von dem vorletzten dadurch, dass der linear polarisierte Laserstrahl über den Polarisationswürfel eingekoppelt wird. Dies hat den Vorteil, dass das Positioniersystem mit dem Kippspiegel KS, der Blende B, dem Positionsdetektor PSD 2 und der Flüssigkeitslinse FL 2 kleiner ist und damit schneller und genauer positioniert werden kann. Die Laserdiode und die Flüssigkeitslinse FL 1 sind nicht beweglich.

Der linear polarisierte Laserdiodenstrahl wird vom Polarisationswürfel zum Kippspiegel KS hin reflektiert. Die Polarisationsrichtung wird durch den Polarisationswürfel nicht geändert. Sie bleibt parallel zur Zeichenebene. Die λ/4-Platte erzeugt zirkular polarisiertes Licht. Bei senkrechtem Einfall auf den Kippspiegel KS wird zirkular polarisiertes Licht reflektiert, das lediglich die Drehrichtung um 180° geändert hat. Die λ/4-Platte wandelt das Licht in linear polarisiertes Licht um. Dabei wird die Schwingungsrichtung des Lichtes um 90° zum linear polarisierten Laserdiodenlicht gedreht. Die Schwingungsrichtung ist nun senkrecht zur Zeichenebene. Der Polarisationswürfel lässt das Licht mit dieser Schwingungsrichtung ohne Reflektion zum Auge durch.

Fällt das zirkular polarisierte Licht unter einem Winkel auf den Kippspiegel, entsteht elliptisch polarisiertes Licht, da beide Anteile (senkrecht und parallel) unterschiedlich reflektiert werden. Das vom Kippspiegel KS kommende, elliptisch polarisierte Licht wird durch die λ/4-Platte in seinen beiden Anteilen (senkrecht und parallel) verändert. Das zur Zeichenebene senkrecht polarisierte Licht passiert den Strahlteiler zum Auge hin ohne Reflektionen. Das zur Zeichenebene parallel polarisierte Licht wird vom Strahlteiler zur Laserdiode hin reflektiert. Mit einem zusätzlichen Spiegel und einem Positionsdetektor können die Position und die Lichtleistung des Laserstrahles erfasst werden.

Da bei schrägem Einfall des Lichtstrahles auf den Kippspiegel KS dasjenige Licht, welches das Auge erreicht, durch den Kippspiegel KS in Abhängigkeit vom Einfallswinkel moduliert wird, muss die Lichtleistung des Laserstrahles durch die Steuerung in Abhängigkeit vom Kippwinkel vorab moduliert werden. So erreicht man einen Messstrahl, dessen Lichtleistung vom Kippwinkel unabhängig ist.

### BEZUGSZEICHENLISTE

- A: Auge
- B: Blende
- BK 1, BK 2: Beobachtungskamera
- E: Einblicköffnung
- FL 1, FL 2: Flüssigkeitslinse
- KS: Kippspiegel
- L 1 bis L 6: Linse
- LD 1: Laserdiode
- LD 2: Bearbeitungslaser
- PM 1 bis PM 4: Piezomotor
- PSD 1 bis PSD 4: Positionsdetektor oder Kamera
- SC: Scanner
- S 1 bis S 5: Strahlteiler
- SL: Streulichtscheibe
- V 1, V 2: Verschluss

## Patentansprüche

1. Vorrichtung zum Messen und zur chirurgischen Korrektur von Abbildungsfehlern im menschlichen Auge, bei der das optische System des Auges (A) mit einem Lichtstrahl einer Laserdiode (LD 1) flächendeckend abtastbar und die reflektierten Lichtstrahlen messbar und auswertbar sind, wobei in den Mess- bzw. Abbildungsstrahlengang zwischen der Laserdiode (LD 1) und dem Auge (A) ein erster Strahlteiler (S 1) eingefügt ist, über den ein Lichtstrahl eines Bearbeitungslasers (LD 2) für eine chirurgische Korrektur der Hornhaut und/oder der Linse und/oder der Netzhaut des Auges (A) einkoppelbar ist, wobei ferner in den Messstrahlengang zwischen der Laserdiode (LD 1) und dem ersten Strahlteiler (S 1) ein Kippspiegel (KS) eingefügt ist, mit dem der Messstrahl zum flächendeckenden Abtasten des optischen Systems des Auges (A) mit Frequenzen von 150 Hz bis 32 kHz zweidimensional ablenkbar ist, und in den Messstrahlengang zwischen dem Kippspiegel (KS) und dem ersten Strahlteiler (S 1) eine erste Linse (L 1) eingefügt ist, **dadurch gekennzeichnet, dass** der Abstand der Linse zu dem Kippspiegel (KS) zum Vorkompensieren des Messstrahles durch einen ersten Piezomotor (PM 1) veränderbar ist.

2. Vorrichtung nach Anspruch 1, **dadurch gekennzeichnet, dass** der in den Messstrahlengang zwischen der Laserdiode (LD 1) und dem ersten Strahlteiler (S 1) eingefügte Kippspiegel (KS) als 2 D Mikro-Scanner-Spiegel ausgebildet ist, mit dem der Messstrahl zum flächendeckenden Abtasten des optischen Systems des Auges (A) mit Frequenzen von 150 Hz bis 32 kHz zweidimensional ablenkbar ist.

3. Vorrichtung nach Anspruch 1 oder 2, **dadurch gekennzeichnet, dass** in den Messstrahlengang zwischen der Laserdiode (LD 1) und dem Kippspiegel (KS) eine erste elektrisch steuerbare Flüssigkeitslinse (FL 1) eingefügt ist, mit der die Divergenz und der Durchmesser des Messstrahles einstellbar sind.

4. Vorrichtung nach einem der Ansprüche 1 bis 3, **dadurch gekennzeichnet, dass** in den Mess- bzw. Abbildungsstrahlengang zwischen der ersten Linse (L 1) und dem ersten Strahlteiler (S 1) ein zweiter Strahlteiler (S 2) eingefügt ist, über den ein Teil des Messstrahles auskoppelbar und einem ersten Positionsdetektor (PSD 1) oder einer schnellen, empfindlichen ersten Kamera mit oder ohne Bildverstärker zuführbar ist, der bzw. die den gleichen Abstand von dem Strahlteiler (S 2) hat wie das optische System des Auges (A) und mit der bzw. dem die Position des auf der Hornhaut des Auges (A) auftreffenden Messstrahles erfassbar ist.

5. Vorrichtung nach Anspruch 4, **dadurch gekennzeichnet, dass** in den Mess- bzw. Abbildungsstrahlengang zwischen dem zweiten Strahlteiler (S 2) und dem ersten Strahlteiler (S 1) ein dritter Strahlteiler (S 3) eingefügt ist, mit dem der Strahlengang der von der Netzhaut des Auges (A) gestreuten Abbildungsstrahlen aufteilbar ist, wobei ein erster Teil der Abbildungsstrahlen zum Begrenzen des Raumwinkels des Abbildungsstrahlenganges über eine zweite Linse (L 2) und eine Blende (B) sowie zum Messen der Brechkraft des optischen Systems des Auges (A) über eine zweite elektrisch steuerbare Flüssigkeitslinse (FL 2) zu einem zweiten Positionsdetektor (PSD 2) oder einer schnellen, empfindlichen zweiten Kamera mit oder ohne Bildverstärker zuführbar ist, wobei der Abbildungsstrahl mit der zweiten Flüssigkeitslinse (FL 2) an den zweiten Positionsdetektor (PSD 2) bzw. an die zweite Kamera anpassbar ist, während ein zweiter Teil der Abbildungsstrahlen einer ersten Beobachtungskamera (BK 1) zuführbar ist, die mit einem Gerät zum Aufzeichnen von Augen- bzw. Blickbewegungen einer Testperson ausgerüstet oder wahlweise durch weitere optische Geräte ersetzbar ist, wie beispielsweise durch ein Mikroskop oder ein Schichtdickenmessgerät.

6. Vorrichtung nach Anspruch 5, **dadurch gekennzeichnet, dass** in den Messstrahlengang zwischen der ersten Linse (L 1) und dem zweiten Strahlenteiler (S 2) ein vierter Strahlenteiler (S 4) eingefügt ist, der den zweiten Teil der von dem dritten und dem zweiten Strahlenteiler (S 3, S 2) reflektierten Abbildungsstrahlen zum Messen des Abstandes zwischen dem Einblick (E) des Messgerätes und dem Auge (A) und zum Überprüfen des Krümmungsradius der Hornhaut des Auges (A) zu einer zweiten Beobachtungskamera (BK 2) mit telezentrischem Objektiv umlenkbar ist.

7. Vorrichtung nach Anspruch 5 oder 6, **dadurch gekennzeichnet, dass** das Auge (A) mit einem über den Kippspiegel (KS) in Form eines Ringes oder eines blinkenden Punktes eingeblendeten Sehzeichen fixierbar ist, wobei der Durchmesser des Ringes bzw. des Punktes mit der ersten Flüssigkeitslinse (FL 1) und dem Kippspiegel (KS) einstellbar ist.

8. Vorrichtung nach einem der Ansprüche 5 bis 7, **dadurch gekennzeichnet, dass** der zweite Positionsdetektor (PSD 2) bzw. die zweite Kamera zum Anpassen des Abbildungsstrahles durch einen zweiten Piezomotor (PM 2) axial verschiebbar ist.

9. Vorrichtung nach einem der Ansprüche 5 bis 8, **dadurch gekennzeichnet, dass** in den Mess- bzw. Abbildungsstrahlengang zwischen dem zweiten Strahlenteiler (S 2) und dem ersten Positionsdetektor (PSD 1) bzw. der ersten Kamera ein fünfter Strahlteiler (S 5) eingefügt ist, mit dem ein Teil des Messstrahles auskoppelbar und über einen ersten Verschluss (V 1) zum Selbstkalibrieren einem Kunstauge zuführbar ist, das den gleichen Abstand von dem zweiten Strahlteiler (S 2) hat wie das optische System des Auges (A) und aus einer dritten Linse (L 3), einer durch einen dritten Piezomotor (PM 3) axial verschiebbaren Streulichtscheibe (SL) und einem durch einen vierten Piezomotor (PM 4) axial verschiebbaren dritten Positionsdetektor (PSD 3) oder einer schnellen, empfindlichen dritten Kamera mit oder ohne Bildverstärker besteht.

10. Vorrichtung nach Anspruch 9, **dadurch gekennzeichnet, dass** die Verzerrungen der elektrischen Signale des ersten, des zweiten und des dritten Positionsdetektors (PSD 1, PSD 2, PSD 3) über elektronische Entzerrerschaltungen oder softwaremäßig kompensierbar und verstärkbar sind, wobei die lokale Brechkraft des optischen Systems des Auges (A) durch Differenzbildung zwischen dem Messwert des zweiten Positionsdetektors (PSD 2) und dem abgespeicherten Sollwert ermittelbar ist.

11. Vorrichtung nach Anspruch 9, **dadurch gekennzeichnet, dass** die lokale Brechkraft des optischen Systems des Auges (A) durch Differenzbildung zwischen den Messwerten des zweiten und des dritten Positionsdetektors (PSD 2, PSD 3) bzw. der zweiten und der dritten Kamera ermittelbar ist.

12. Vorrichtung nach einem der Ansprüche 5 bis 11, **dadurch gekennzeichnet, dass** statt der ersten Linse (L 1) und der zweiten Linse (L 2) eine einzige Linse in den Mess- bzw. Abbildungsstrahlengang zwischen dem zweiten Strahlteiler (S 2) und dem dritten Strahlteiler (S 3) eingefügt ist.

13. Vorrichtung nach einem der Ansprüche 5 bis 12, **dadurch gekennzeichnet, dass** die Position des Kippspiegels (KS) statt durch den ersten Positionsdetektor (PDS 1) bzw.
die erste Kamera durch an dem Kippspiegel (KS) angebrachte Sensoren ermittelbar ist.

14. Vorrichtung nach einem der Ansprüche 5 bis 13, **dadurch gekennzeichnet, dass** in den Strahlengang des Bearbeitungsstrahles zwischen dem Bearbeitungslaser (LD 2) und dem ersten Strahlteiler (S 1) zwei Scanner (SC) zum Ablenken des Bearbeitungsstrahles in zwei rechtwinklig zueinander verlaufenden Richtungen (X, Y) sowie eine vierte Linse (L 4) zum Einstellen des Arbeitsabstandes zwischen dem Bearbeitungslaser (LD 2) und dem Auge (A) eingefügt sind.

15. Vorrichtung nach einem der Ansprüche 5 bis 14, **dadurch gekennzeichnet, dass** mit dem ersten Strahlteiler (S 1) einen Teil des Bearbeitungsstrahles auskoppelbar und einem vierten Positionsdetektor (PSD 4) oder einer schnellen, empfindlichen vierten Kamera mit oder ohne Bildverstärker zuführbar ist, der bzw. die den gleichen Abstand von dem ersten Strahlteiler (S 1) hat wie das optische System des Auges (A) und mit dem bzw. der die Energie, die Pulsbreite und die Leistung des Bearbeitungslasers (LD 2) überwachbar sind und außerdem gleichzeitig die aktuelle Position des Bearbeitungsstrahles messbar und zum Justieren des Bearbeitungs-, Mess- und Abbildungsstrahlenganges verwendbar ist.

16. Vorrichtung nach einem der Ansprüche 5 bis 15, **dadurch gekennzeichnet, dass** in den Mess- bzw. Abbildungsstrahlengang zwischen dem ersten und dem dritten Strahlenteiler (S 1, S 3) zur Anpassung des Messabstandes an den Bearbeitungsabstand eine fünfte und eine sechste Linse (L 5, L 6) eingefügt sind, deren Abbildungsverhältnis auf 1:1 eingestellt ist.

17. Vorrichtung nach einem der Ansprüche 5 bis 16, **dadurch gekennzeichnet, dass** zum Messen der lokalen Brechkraft von beliebigen Linsen die erste Linse (L 1) in ihrem Durchmesser an den Durchmesser der zu messenden Linsen angepasst ist.

18. Vorrichtung nach einem der Ansprüche 5 bis 17, **dadurch gekennzeichnet, dass** die Blende (B), die zweite Flüssigkeitslinse (FL 2) und der zweite Positionsdetektor (PSD 2) gemeinsam mit der Laserdiode (LD 1), der ersten Flüssigkeitslinse (FL 1), dem Kippspiegel (KS) und dem ersten Piezomotor (PM 1) auf einem Positioniersystem derart montiert sind, dass die optische Achse des durch die Öffnung der Blende (B) eintretenden Abbildungsstrahles parallel zu der optischen Achse des den Kippspiegel (KS) verlassenden Messstrahles verläuft, wobei das Positioniersystem durch einen weiteren Piezomotor in Richtung der optischen Achsen verschiebbar ist.

19. Vorrichtung nach einem der Ansprüche 5 bis 17, **dadurch gekennzeichnet, dass** die Blende (B), die zweite Flüssigkeitslinse (FL 2) und der zweite Positionsdetektor (PSD 2) gemeinsam mit dem Kippspiegel (KS) auf einem Positioniersystem derart montiert sind, dass die optische Achse des durch die Öffnung der Blende (B) eintretenden Abbildungsstrahles parallel zu der optischen Achse des den Kippspiegel (KS) verlassenden Messstrahles verläuft, wobei der von der Laserdiode (LD 1) kommende Messstrahl über dieselbe optische Achse den Kippspiegel (KS) erreicht, über die der abgelenkte Messstrahl den Kippspiegel (KS) verlässt, und wobei das Positioniersystem durch einen weiteren Piezomotor in Richtung der optischen Achsen verschiebbar ist.

20. Vorrichtung nach Anspruch 19, **dadurch gekennzeichnet, dass** der Messstrahl nach Verlassen der Laserdiode (LD 1) über die erste Flüssigkeitslinse (FL 1) und nach Reflexion an einem Polarisationswürfel über eine λ/4-Platte über die optische Achse durch den Kippspiegel (KS) ablenkbar ist und über die optische Achse, über die λ/4-Platte, über den Strahlteilerwürfel und über eine Linse dem zu messenden Auge (A) zuführbar ist, von wo aus die an der Netzhaut gestreuten Abbildungsstrahlen über diese Linse und nach Reflexion an dem Polarisationswürfel über eine weitere Linse und einen reflektierenden Strahlenteiler durch die Blende (B) und die Flüssigkeitslinse (FL 2) dem Positionsdetektor (PSD 2) zuführbar sind, während die an der Hornhaut des Auges (A) reflektierten Strahlen auf demselben Weg wie die Abbildungsstrahlen nach Passieren des Strahlenteilers über eine weitere Linse der Beobachtungskamera (BK 2) mit telezentrischem Objektiv zuführbar sind.

## Claims

1. A device for measuring and for surgical correction of imaging errors in the human eye, in the case of which the optical system of the eye (A) is scanned with a light beam of a laser diode (LD 1) in an area-wide fashion, and the reflected light beams are measured and evaluated, wherein inserted into the measuring and/or imaging beam path between the laser diode (LD 1) and the eye (A) is a first beam splitter (S 1) via which a light beam of a treatment laser (LD 2) for a surgical correction of the cornea and/or lens and/or the retina of the eye (A) can be coupled in, wherein there is inserted into the measuring beam path between the laser diode (LD 1) and the first beam splitter (S 1) a tilting mirror (KS) with the aid of which the measuring beam can be deflected in two dimensions for the purpose of area-wide scanning of the optical system of the eye (A) with frequencies from 150 Hz to 32 kHz and inserted into the measuring beam path between the tilting mirror (KS) and the first beam splitter (S 1) is a first lens (L 1), **characterized in that** the distance of the lens from the tilting mirror (KS) can be varied for precompensation of the measuring beam by a first piezomotor (PM 1).

2. The device as claimed in claim 1, **characterized in that** there is inserted as a tilting mirror (KS) into the measuring beam path between the laser diode (LD 1) and the first beam splitter (S 1) a 2 D microscanner mirror with the aid of which the measuring beam can be deflected in two dimensions for the purpose of area-wide scanning of the optical system of the eye (A) with frequencies from 150 Hz to 32 kHz.

3. The device as claimed in claim 1 or 2, **characterized in that** inserted into the measuring beam path between the laser diode (LD 1) and the tilting mirror (KS) is a first electrically controllable liquid lens (FL 1) with the aid of which the divergence and the diameter of the measuring beam can be set.

4. The device as claimed in one of the claims 1 to 3, **characterized in that** inserted into the measuring and/or imaging beam path between the first lens (L 1) and the first beam splitter (S 1) is a second beam splitter (S 2), which decouples a portion of the measuring beam and feeds it to a first position detector (PSD 1) or to a fast, sensitive first camera with or without image intensifier, which detector or camera is at the same distance from the beam splitter (S 2) as the optical system of the eye (A) and detects the position of the measuring beam striking the cornea of the eye (A).

5. The device as claimed in claim 4, **characterized in that** inserted into the measuring and/or imaging beam path between the second beam splitter (S 2) and the first beam splitter (S 1) is a third beam splitter (S 3), which splits the beam path of the imaging beams dispersed by the retina of the eye (A), a first portion of the imaging beams passing for the purpose of delimit the solid angle of the imaging beam path via a second lens (L 2) and an aperture (B), and for the purpose of measuring the refractive power of the optical system of the eye (A) via a second electrically controllable liquid lens (FL 2) to a second position detector (PSD 2) or to a fast, sensitive second camera with or without image intensifier, it being possible to adapt the imaging beam with the second liquid lens (FL 2) to the second position detector (PSD 2) and/or to the second camera, while a second portion of the imaging beams passes to an observation camera (BK 1) that is equipped with a unit for recording eye movements and/or viewing movements of a test subject or can optionally be replaced by further optical units such as, for example, a microscope or a layer thickness measurement unit.

6. The device as claimed in claim 5, **characterized in that** inserted into the measuring beam path between the first lens (L 1) and the second beam splitter (S 2) is a fourth beam splitter (S 4), which deflects the second portion of the imaging beams reflected by the third and the second beam splitters (S 3, S 2) to a second observation camera (BK 2) with telecentric lens for the purpose of measuring the distance between the viewer (E) of the measuring unit and the eye (A), and of checking the radius of curvature of the cornea of the eye (A).

7. The device as claimed in claim 5 or 6, **characterized in that** the eye (A) can be fixed with the aid of a optotype inserted in the form of a ring or a flashing point via the tilting mirror (KS), it being possible to set the diameter of the ring or the point with the aid of a first liquid lens (FL 1) and the tilting mirror (KS).

8. The device as claimed in one of claims 5 to 7, **characterized in that** the second position detector (PSD 2) or the second camera can be axially displaced for adaptation of the imaging beam by a second piezomotor (PM 2).

9. The device as claimed in one of claims 5 to 8, **characterized in that** inserted into the measuring and/or imaging beam path between the second beam splitter (S 2) and the first position detector (PSD 1) or the first camera is a fifth beam splitter (S 5), which couples out a portion of the measuring beam and feeds it via a first shutter (V 1) for self calibration to an artificial eye that is at the same distance from the second beam splitter (S 2) as the optical system of the eye (A) and consists of a third lens (L 3), a diffuser plate (SL) that can be axially displaced by a third piezomotor (PM 3), and a third position detector (PSD 3) that can be axially displaced by a fourth piezomotor (PM 4), or a fast, sensitive third camera with or without image intensifier.

10. The device as claimed in claim 9, **characterized in that** the distortions of the electrical signals of the first, the second and the third position detector (PSD 1, PSD 2, PSD 3) are compensated and amplified via electronic equalizing circuits or by means of software, the local refractive force of the optical system of the eye (A) being determined by calculating the difference between the measured value of the second position detector (PSD 2) and the stored desired value.

11. The device as claimed in claim 9, **characterized in that** the local refractive power of the optical system of the eye (A) is determined by calculating the difference between the measured values of the second and the third position detector (PSD 2, PSD 3) or the second and third camera.

12. The device as claimed in one of claims 5 to 11, **characterized in that** instead of the first lens (L 1) and the second lens (L 2), a single lens is inserted into the measuring and/or imaging beam path between the second beam splitter (S 2) and the third beam splitter (S 3).

13. The device as claimed in one of claims 5 to 12, **characterized in that** instead of being determined by the first position detector (PDS 1) or the first camera, the position of the tilting mirror (KS) is determined by sensors mounted on the tilting mirror (KS).

14. The device as claimed in one of claims 5 to 13, **characterized in that** two scanners (SC) for deflecting the treatment beam in two directions (X, Y) running perpendicular to one another, and a fourth lens (L 4) for setting the working distance between the treatment laser (LD 2) and the eye (A) are inserted into the beam path of the treatment beam between the treatment laser (LD 2) and the first beam splitter (S 1).

15. The device as claimed in one of claims 5 to 14, **characterized in that** the first beam splitter (S 1) couples out a portion of the treatment beam and feeds it to a fourth position detector (PSD 4) or a fast, sensitive fourth camera with or without image intensifier, which detector or camera is at the same distance from the first beam splitter (S 1) as the optical system of the eye (A) and measures the energy, the pulse width and the output of the treatment laser (LD 2) and, moreover, simultaneously measures the current position of the treatment beam and serves the purpose of adjusting the treatment, measuring and imaging beam paths.

16. The device as claimed in one of claims 5 to 15, **characterized in that** inserted into the measuring beam path and/or imaging beam path between the first and the third beam splitters (S 1, S 3) for adaptation of the measuring distance to the treatment distance are a fifth and a sixth lens (L 5, L 6) whose scale ratio is set to 1:1.

17. The device as claimed in one of claims 5 to 16, **characterized in that** the diameter of the first lens (L 1) is adapted to the diameter of the lenses to be measured in order to measure the local refractive power of any desired lenses.

18. The device as claimed in one of claims 5 to 17, **characterized in that**, in common with the laser diode (LD 1), the first liquid lens (FL 1), the tilting mirror (KS) and the first piezomotor (PM 1), the aperture (B), the second liquid lens (FL 2) and the second position detector (PSD 2) are mounted on a positioning system in such a way that the optical axis of the imaging beam entering through the opening in the aperture (B) runs parallel to the optical axis of the measuring beam leaving the tilting mirror (KS), it being possible to displace the positioning system in the direction of the optical axes by a further piezomotor.

19. The device as claimed in one of claims 5 to 17, **characterized in that** in common with the tilting mirror (KS), the aperture (B), the second liquid lens (FL 2) and the second position detector (PSD 2) are mounted on a positioning system in such a way that the optical axis of the imaging beam entering through the opening in the aperture (B) runs parallel to the optical axis of the measuring beam leaving the tilting mirror (KS), the measuring beam coming from the laser diode (LD 1) reaching the tilting mirror (KS) via the same optical axis as the deflected measuring beam that leaves the tilting mirror (KS), and it being possible to displace the positioning system in the direction of the optical axes by a further piezomotor.

20. The device as claimed in claim 19, **characterized in that** via the first liquid lens (FL 1) the measuring beam coming from the laser diode (LD 1) strikes a polarization cube, is reflected there and, passing via a λ/4 plate, via the optical axis, reaches the tilting mirror (KS), starting from which the deflected measuring beam, passing via the optical axis, the λ/4 plate, the beam splitter cube and a lens, strikes the eye (A) to be measured, starting from which the imaging beams dispersed at the retina pass via this lens and strike the polarization cube, are reflected there and, via a further lens and a reflecting beam splitter, reach the position detector (PSD 2) through the aperture (B) and the liquid lens (FL 2), while the beams reflected at the cornea of the eye (A) strike the beam splitter on the same path as the imaging beams and are let pass there so as to reach the observation camera (BK 2) with telecentric lens via a further lens.

## Revendications

1. Dispositif pour mesurer et corriger de façon chirurgicale des défauts de représentation dans l'oeil humain, sur lequel le système optique de l'oeil (A) peut être balayé en couvrant la surface avec un faisceau lumineux d'une diode laser (LD 1) et les faisceaux lumineux réfléchis peuvent être mesurés et analysés, un premier diviseur de faisceau (S 1) étant inséré dans la trajectoire du faisceau de mesure ou du faisceau de représentation entre la diode laser (LD 1) et l'oeil (A), diviseur par lequel un faisceau lumineux d'un laser de traitement (LD 2) peut être injecté pour une correction chirurgicale de la cornée et/ou de la lentilles et/ou de la rétine de l'oeil (A), un miroir basculant (KS) étant inséré également dans la trajectoire du faisceau de mesure entre la diode laser (LD 1) et le premier diviseur de faisceau (S 1), miroir avec lequel le faisceau de mesure peut être dévié dans deux dimensions pour le balayage avec couverture de surface du système optique de l'oeil (A) avec des fréquences allant de 150 Hz à 32 kHz, et
une première lentille (L 1) étant insérée dans la trajectoire du faisceau de mesure entre le miroir basculant (KS) et le premier diviseur de faisceau (S1),
**caractérisé en ce que** la distance de la lentille au miroir basculant (KS) peut être modifiée par un premier moteur piézoélectrique (PM 1) pour la précompensation du faisceau de mesure.

2. Dispositif selon la revendication 1, **caractérisé en ce que** le miroir basculant (KS) inséré dans la trajectoire du faisceau de mesure entre la diode laser (LD 1) et le premier diviseur de faisceau (S 1) est conçu sous la forme d'un miroir de microscanner à deux dimensions, avec lequel le faisceau de mesure peut être dévié dans deux dimensions pour le balayage avec couverture de surface du système optique de l'oeil (A) avec des fréquences allant de 150 Hz à 32 kHz.

3. Dispositif selon la revendication 1 ou 2, **caractérisé en ce qu'**une première lentille à liquide (FL 1) pouvant être commandée électriquement est insérée dans la trajectoire du faisceau de mesure entre la diode laser (LD 1) et le miroir basculant (KS), lentille avec laquelle la divergence et le diamètre du faisceau de mesure peuvent être réglés.

4. Dispositif selon l'une quelconque des revendications 1 à 3, **caractérisé en ce que** dans la trajectoire du faisceau de mesure ou la trajectoire du faisceau de représentation, entre la première lentille (L 1) et le premier diviseur de faisceau (S1), est inséré un deuxième diviseur de faisceau (S 2), par lequel une partie du faisceau de mesure peut être dissociée et peut être amenée à un premier détecteur de position (PSD 1) ou à une première caméra rapide et sensible, équipée ou non d'amplificateur d'image, lequel détecteur ou laquelle caméra est à la même distance du diviseur de faisceau (S 2) que le système optique de l'oeil (A) et avec laquelle ou lequel la position du faisceau de mesure arrivant sur la cornée de l'oeil (A) peut être enregistrée.

5. Dispositif selon la revendication 4, **caractérisé en ce que** dans la trajectoire du faisceau de mesure ou la trajectoire du faisceau de représentation, entre le deuxième diviseur de faisceau (S 2) et le premier diviseur de faisceau (S 1), est inséré un troisième diviseur de faisceau (S 3), avec lequel la trajectoire des faisceaux de représentation diffusés par la rétine de l'oeil (A) peut être divisée, une première partie des faisceaux de représentation pouvant être amenée par une deuxième lentille (L 2) et un diaphragme (B) pour la délimitation de l'angle dans l'espace de la trajectoire du faisceau de représentation et par une deuxième lentille à liquide (FL 2) pouvant être commandée électriquement pour la mesure du pouvoir réfringent du système optique de l'oeil (A) à un deuxième détecteur de position (PS 2) ou à une deuxième caméra rapide et sensible, équipée ou non d'amplificateur d'image, le faisceau de représentation pouvant être adapté avec la deuxième lentille à liquide (FL 2) au deuxième détecteur de position (PSD 2) ou à la deuxième caméra, alors qu'une seconde partie des faisceaux de représentation peut être amenée à une première caméra d'observation (BK 1), qui est équipée d'un appareil pour l'enregistrement des mouvements de l'oeil ou du regard d'un sujet ou peut être remplacée au choix par d'autres appareils optiques, par exemple par un microscope ou un appareil de mesure d'épaisseur de couche.

6. Dispositif selon la revendication 5, **caractérisé en ce que** dans la trajectoire du faisceau de mesure, entre la première lentille (L 1) et le deuxième diviseur de faisceau (S 2), est inséré un quatrième diviseur de faisceau (S4) qui peut dévier la seconde partie des faisceaux de représentation réfléchis par le troisième et le deuxième diviseur de faisceau (S 3, S 2) pour mesurer la distance entre le viseur (E) de l'appareil de mesure et l'oeil (A) et pour contrôler le rayon de courbure de la cornée de l'oeil (A) par rapport à une deuxième caméra d'observation (BK 2) avec objectif télécentrique.

7. Dispositif selon la revendication 5 ou 6, **caractérisé en ce que** l'oeil (A) peut être fixé avec une mire insérée par l'intermédiaire du miroir basculant (KS) sous la forme d'une bague ou d'un point clignotant, le diamètre de la bague ou du point pouvant être réglé avec la première lentille à liquide (FL 1) et le miroir basculant (KS).

8. Dispositif selon l'une quelconque des revendications 5 à 7, **caractérisé en ce que** le deuxième détecteur de position (PSD 2) ou la deuxième caméra peut être déplacé(e) axialement par un deuxième moteur piézoélectrique (PM 2) pour l'adaptation du faisceau de représentation.

9. Dispositif selon l'une quelconque des revendications 5 à 8, **caractérisé en ce que** dans la trajectoire du faisceau de mesure ou la trajectoire du faisceau de représentation, entre le deuxième diviseur de faisceau (S 2) et le premier détecteur de position (PSD 1) ou la première caméra, est inséré un cinquième diviseur de faisceau (S 5), avec lequel une partie du faisceau de mesure peut être dissociée et peut être amenée par une première fermeture (V 1) pour l'autocalibrage à un oeil artificiel, qui est à la même distance du deuxième diviseur de faisceau (S 2) que le système optique de l'oeil (A) et se compose d'une troisième lentille (L 3), d'un disque diffusant (SL) pouvant être déplacé axialement par un troisième moteur piézoélectrique (PM 3) et d'un troisième détecteur de position (PSD 3) pouvant être déplacé axialement par un quatrième moteur piézoélectrique (PM 4) ou d'une troisième caméra rapide et équipée ou non d'amplificateur d'image.

10. Dispositif selon la revendication 9, **caractérisé en ce que** les distorsions des signaux électriques du premier, du deuxième et du troisième détecteur de position (PSD 1, PSD 2, PSD 3) peuvent être compensées et amplifiées par des circuits électroniques correcteurs de distorsion ou par un logiciel, le pouvoir réfringent local du système optique de l'oeil (A) pouvant être déterminé par la soustraction entre la valeur de mesure du deuxième détecteur de position (PSD 2) et la valeur de consigne mémorisée.

11. Dispositif selon la revendication 9, **caractérisé en ce que** le pouvoir réfringent local du système optique de l'oeil (A) peut être déterminé par la détermination de la différence entre les valeurs de mesure du deuxième et du troisième détecteur de position (PSD 2, PDS 3) et celles de la deuxième et de la troisième caméra.

12. Dispositif selon l'une quelconque des revendications 5 à 11, **caractérisé en ce que**, au lieu de la première lentille (L1) et de la deuxième lentille (L 2), une lentille unique est insérée dans la trajectoire du faisceau de mesure ou la trajectoire du faisceau de représentation entre le deuxième diviseur de faisceau (S 2) et le troisième diviseur de faisceau (S 3).

13. Dispositif selon l'une quelconque des revendications 5 à 12, **caractérisé en ce que** la position du miroir basculant (KS) peut être déterminée par des capteurs placés sur le miroir basculant (KS) au lieu d'être déterminée par le premier détecteur de position (PDS 1) ou la première caméra.

14. Dispositif selon l'une quelconque des revendications 5 à 13, **caractérisé en ce que** dans la trajectoire du faisceau de traitement, entre le laser de traitement (LD 2) et le premier diviseur de faisceau (S 1), sont insérés deux scanners (SC) pour la déviation du faisceau de traitement dans deux directions (X, Y) agencées perpendiculairement entre elles et une quatrième lentille (L 4) pour le réglage de la distance de travail entre le laser de traitement (LD 2) et l'oeil (A).

15. Dispositif selon l'une quelconque des revendications 5 à 14, **caractérisé en ce que**, avec le premier diviseur de faisceau (S 1), une partie du faisceau de traitement peut être dissociée et amenée à un quatrième détecteur de position (PSD 4) ou à une quatrième caméra rapide et sensible avec ou sans amplificateur d'image, lequel détecteur ou laquelle caméra est à la même distance du premier diviseur de faisceau (S 1) que le système optique de l'oeil (A) et avec lequel ou laquelle l'énergie, l'amplitude d'impulsion et la puissance du laser de traitement (LD 2) peuvent être contrôlées et d'autre part la position actuelle du faisceau de traitement peut être mesurée simultanément et utilisée pour l'ajustement de la trajectoire du faisceau de traitement, du faisceau de mesure et du faisceau de représentation.

16. Dispositif selon l'une quelconque des revendications 5 à 15, **caractérisé en ce que** dans la trajectoire du faisceau de mesure ou du faisceau de représentation, entre le premier et le troisième diviseur de faisceau (S1, S3), sont insérées une cinquième et une sixième lentille (L 5, L 6) pour l'adaptation de la distance de mesure à la distance de traitement, lentilles dont le rapport de représentation est réglé à 1:1.

17. Dispositif selon l'une quelconque des revendications 5 à 16, **caractérisé en ce que**, pour la mesure du pouvoir réfringent local de lentilles quelconques, la première lentille (L 1) est adaptée dans son diamètre au diamètre des lentilles à mesurer.

18. Dispositif selon l'une quelconque des revendications 5 à 17, **caractérisé en ce que** le diaphragme (B), la deuxième lentille à liquide (FL 2) et le deuxième détecteur de position (PSD 2) sont montés conjointement avec la diode laser (LD 1), la première lentille à liquide (FL 1), le miroir basculant (KS) et le premier moteur piézoélectrique (PM 1) sur un système de positionnement, de telle sorte que l'axe optique du faisceau de représentation entrant par l'ouverture du diaphragme (B) est agencé parallèlement à l'axe optique du faisceau de mesure quittant le miroir basculant (KS), le système de positionnement pouvant être déplacé par un autre moteur piézoélectrique en direction des axes optiques.

19. Dispositif selon l'une quelconque des revendications 5 à 17, **caractérisé en ce que** le diaphragme (B), la deuxième lentille à liquide (FL 2) et le deuxième détecteur de position (PSD 2) sont montés conjointement avec le miroir basculant (KS) sur un système de positionnement, de telle sorte que l'axe optique du faisceau de représentation entrant par l'ouverture du diaphragme (B) est agencé parallèlement à l'axe optique du faisceau de mesure quittant le miroir basculant (KS), le faisceau de mesure arrivant de la diode laser (LD 1) atteignant le miroir basculant (KS) par le même axe optique, axe par lequel le faisceau de mesure dévié quitte le miroir basculant (KS), et le système de positionnement pouvant être déplacé par un autre moteur piézoélectrique en direction des axes optiques.

20. Dispositif selon la revendication 19, **caractérisé en ce que** le faisceau de mesure peut être dévié, après avoir quitté la diode laser (LD 1), par la première lentille à liquide (FL 1) et, après réflexion sur un cube de polarisation, par une plaque quart d'onde via l'axe optique, par le miroir basculant (KS) et peut être amené via l'axe optique, par la plaque quart d'onde, par le cube diviseur de faisceau et par une lentille à l'oeil (A) à mesurer, à partir duquel les faisceaux de représentation diffusés sur la rétine peuvent être amenés par cette lentille et, après réflexion sur le cube de polarisation, par l'intermédiaire d'une autre lentille et d'un diviseur de faisceau réfléchissant par le diaphragme (B) et la lentille à liquide (FL 2) au détecteur de position (PSD 2), alors que les faisceaux réfléchis sur la cornée de l'oeil (A) peuvent être amenés sur le même trajet que les faisceaux de représentation, après être passés par le diviseur de faisceau, au moyen d'une autre lentille à la caméra d'observation (BK 2) avec objectif télécentrique.
